# EUROPEAN PATENT SPECIFICATION

(11) **EP 1 161 146 B1**
(45) Date of publication and mention of the grant of the patent: **25.05.2005**
(21) Application number: 00993301.1
(22) Date of filing: 13.12.2000
(51) Int. Cl.: A01N 31/02, A01N 33/12, A01N 31/14, A01N 47/44

(54) **DEEP PENETRATING ANTIMICROBIAL COMPOSITIONS**
TIEFEINDRINGENDE ANTIMIKROBIELLE ZUSAMMENSETZUNGEN
COMPOSITIONS ANTIMICROBIENNES A FORTE PENETRATION

(30) Priority: 13.12.1999 US 460014
(43) Date of publication of application: 12.12.2001
(73) Proprietor: ETHICON, INC., Somerville, NJ 08876 (US)
(72) Inventor: JAMPANI, Hanuman, Bridgewater, NJ 08807 (US); HOLLY, Thomas, F., Arlington, TX 76014 (US); NEWMAN, Anthony, Wayne, Fort Worth, TX 76111 (US); NEWMAN, Jerry, L., Arlington, TX 76016 (US)
(74) Representative: Mercer, Christopher Paul
(86) International application number: PCT/US2000/033689
(87) International publication number: WO 2001/041567

(56) References cited:
- EP-A- 0 930 065
- EP-A- 0 937 394
- WO-A-93/07250
- WO-A-97/31533
- US-A- 4 464 293
- DATABASE WPI Section Ch, Week 199441 Derwent Publications Ltd., London, GB; Class D22, AN 1994-329854 XP002166681 & JP 06 256106 A (MILDO SANGYO KK), 13 September 1994 (1994-09-13)

## Description

This invention is related to antimicrobial compositions which provide instant and long-lasting antimicrobial activity.

The normal skin flora consists of both resident and transient populations of bacteria. It is thought that chronic exposure to pathogenic organisms in a hospital environment can lead to their becoming part of the resident flora of the stratum corneum. In a healthcare setting, nosocomial infections are mostly spread through the more loosely-attached transient flora. Most transient organisms can be rinsed away mechanically by simple handwashing with a non-antimicrobial soap. In surgical environments, it is also critical to reduce the resident populations of bacteria, which are frequently pathogenic. The dramatic reduction of these deeper and more adherent bacteria requires potent antiseptics, or chemical disinfection. Residual efficacy depends on penetration, release and retention of antimicrobial agents into the stratum corneum to prevent recolonization of bacteria.

The most commonly used active ingredients in today's surgical scrubs are chlorhexidine gluconate (CHG) and iodophors, such as povidone-iodine(PVP-Iodine). CHG exhibits broad-spectrum antimicrobial activity and extended antimicrobial persistence, by binding to young epithelial cells for an extended time. While considered to be generally safe, allergic reactions do occur. The antimicrobial activity of PVP-Iodine is also quite good, but its persistence is poor, and is easily inactivated by blood and organic materials. The oxidising nature of iodine also leads to the typical harshness of iodophor types of scrubs.

There are only two Category I active ingredients specifically mentioned in the monograph for Surgical Hand Scrubs (21CFR 333.414 Vol. 59, No. 116), alcohol and iodine.

The most safe, rapid-acting and broad spectrum antimicrobial is undoubtedly alcohol. It chemically dissolves and disrupts cell walls of both gram positive and negative bacteria. Its residual activity is extremely limited but the log₁₀ reduction of bacteria is so severe that populations cannot re-establish themselves for several hours after application. Currently, alcoholic hand disinfection is more universally used in surgical wards in Europe than in the United States. Because of its strong antiseptic action and reasonably good skin tolerance when properly formulated, high alcoholic products are also becoming well accepted in the U.S., as shown by the recent surge of popularity of antiseptic hand gels in the consumer and healthcare provider markets.

EP-A-0937394 relates to an antimicrobial composition comprising a) an antimicrobial selected from the group consisting of greater than 30% by volume alcohol and an effective amount of triclosan; and b) an effective amount of phenoxy ethanol, benzaolkonium chloride or benzetheonium chloride; and cocophosphatidyl-dimonium chloride. The composition may be a gelled composition as the result of the presence of thickening agents or may be emulsified with a surfactant.

EP-A-0930065 discloses an antimicrobial composition which possesses the feel and moisturising attributes of a hand cream or lotion and comprises at least about 40 percent by weight alcohol, an effective amount of a carbomer polymer, an effective amount of a cationic antimicrobial compound, and water; the composition having a viscosity of greater than about 5,000 centipoise.

WO 97/31533 discloses an antimicrobial composition, particularly for antiseptics and/or disinfection, which includes at least a combination of one or more biguanide salts and one or more aromatic alcohols, and may contain gelling agents, agents for softening/moisturising and/or surfactants.

WO 93/07250 discloses an antiseptic cleansing composition comprising an alkanol and an aqueous concentrate of a surfactant and, optionally, a biocide. The associated method for cleansing skin comprises applying this composition onto the skin allowing the alkanol in the composition on the skin to evaporate whereby the skin is substantially dry, applying a small amount of water to the substantially dry skin, washing the skin under foam generation and finally rinsing the skin.

JP-A-6256106 discloses a germicidal composition containing germicidal components containing a quaternary ammonium salt, 2-phenoxyethanol, ethanol and/or isopropyl alcohol in an amount of 15 to 25wt.% based on the whole composition with the content of ethanol and/or isopropyl alcohol in the germicidal components being >=50wt.%.

Accordingly, there is a need for an efficacious, convenient, surgical handwash, which will exhibit excellent instantaneous antibacterial kill as well as persistent antimicrobial activity equal to or surpassing the current state of the art. The improved antimicrobial composition should be achieved without the known drawbacks and disadvantages such as requiring a lengthy surgical scrub application procedure, requiring use of scrub brushes which are harsh to the skin due to mechanical abrasion; being drying to the skin; causing the possibility of allergic reaction such as with CHG; or causing the possibility of irritation or sensitization particularly when using CHG or iodophors.

That is, improved antimicrobial compositions should be non-irritating, moisturizing, and should leave a protective barrier on the skin after washing, possibly extending to latex protein blocking ability. Acceptability of such a product would be superior to surgeons and health care workers and thus increase compliance with handwashing protocols. The invention (product) is intended to replace traditional preoperative scrubs containing CHG, hexachlorophene, iodophors, and parachlorometaxylenol (chloroxylenol).

### SUMMARY OF THE INVENTION

This invention relates to an antimicrobial handwash composition as defined in the appendant claims.

In one embodiment, the cationic quaternary ammonium compound is selected from the group consisting of benzalkonium chloride, benzethonium chloride, cetylpyridinium chloride and mixtures thereof, with the surfactant system being a mixture of nonionic, and cationic surfactants and optionally amphoteric surfactants, and with the optional biguanide compound present.

Desirably, the compositions of the invention further comprise an effective amount of a compatible skin conditioning system, the system comprising of skin conditioners and percutaneous enhancers such as glycerin, phenylethyl dimethicone, silicone quaternary compounds (e.g. LAMBENT QUAT AD, available from Lambent Technologies), and propylene glycol.

### DETAILED DESCRIPTION OF PREFERRED EMBODIMENTS OF THE INVENTION

The present invention is directed to antimicrobial compositions comprising a blend of antimicrobial agents and a particular combination of surfactants, the surfactant not including any anionic surfactants.

In the present invention, the antimicrobial compositions of this invention contain alcohol and such alcohol-containing compositions have extremely high antimicrobial effectiveness even when used as a wash-off product. Thus, despite the inclination of those skilled in the art that the wash-off nature of a product is a disadvantage due the active antimicrobial being rinsed away, the compositions of this invention appear to compensate for loss of active antimicrobial due to rinsing by providing enhanced penetrating and depositing properties.

The antimicrobial components of the present invention contain an effective amount of cationic quaternary ammonium compounds, and a surfactant system of nonionic, cationic, and optionally amphoteric surfactants, and desirably a biguanide compound.

Examples of cationic quaternary ammonium compounds include benzalkonium chloride, benzethonium chloride, methylbenzethonium chloride, polymeric ammonium chloride, and bisquaternary ammonium compounds.

Examples of biguanide compounds include chlorhexidine or its derivatives, such as chlorhexidine gluconate, chlorhexidine digluconate, chlorhexidine diacetate, chlorhexidine dihydrochloride and polyhexamethylene biguanide.

Other optional antimicrobial compounds include, alkyl pyridinium salts such as cetylpyridinium chloride; antimicrobial polypeptides such as Nisin (34 amino acid peptide) and of different families such as amphiphilic Cysteine containing beta sheet peptides (Defensins), Cysteine-Disulfide ring peptides (Cyclic dodecapeptide, Ranlexin, Brevinins), Amphiphilic alpha-helix peptides (Magainins, Cecropins), linear peptides with one or two predominant amino acids, Mammalian and Avian disulfide-linked antimicrobial molecules (Human neutrophil peptide, Human defensin, Neutrophil peptide, Macrophage cationic peptide and beta-defensins).

Preferred antimicrobial compounds include benzalkonium chloride and/or benzethonium chloride, polyhexamethylene biguanide, propylene glycol, Coco PG-dimonium chloride phosphate (phospholipid CDM), chlorhexidine gluconate and/or cetyl-pyridinium chloride.

The effective amounts of the foregoing antimicrobial agents will typically be in the following weight ranges, but to those skilled in the art variation in the following ranges may occur but with the benefits of this invention still being achieved: benzalkonium chloride typically 0.02 to 2.0%, preferably 0.05 to 1.0%; most preferably 0.05 to 0.15%, benzethonium chloride typically 0.02 to 5.0%, preferably 0.02 to 1.0%, most preferably 0.05 to 0.12 %; polyhexamethylene biguanide typically 0.01 to 5.0%, preferably 0.02 to 1.0%, most preferably 0.03 to 0.5 %;phenoxyethanol typically 0.1 to 5.0%, preferably 0.2 to 3.0%, most preferably 0.5 to 2.0%; propylene glycol typically 0.1 to 40%, preferably 1.0 to 20.0%, most preferably 5.0 to 15.0%; Coco-PG dimonuim chloride phosphate typically 0.1 to 5.0%, preferably 0.2 to 2.5%, most preferably 0.5 to 2.0%; and cetylpyridinium chloride typically 0.01 to 0.5%; preferably 0.02 to 0.35%, most preferably 0.05 to 0.3%.

In addition to the foregoing antimicrobial agents, the following optional antimicrobial agents may be used: Quaternium-15 (Dowcil-200) typically 0.1 to 1.0%; Boregeamidoprophyl phosphatidyl PG-Dimonium Chloride (Phospholipid GLA) typically 0.1 to 2.0%; Coco PG-dimonuim chloride phosphate (Phospholipid CDM) typically 0.1 to 5.0%; triclosan typically 0.1 to 2.0%; chlorhexidine gluconate typically 0.01 to 5.0%; polyhexamethylenebiguanide hydrochloride typically 0.02 to 5% and methylbenzethonium chloride typically 0.05 to 2.0% by weight.

The surfactant system useful in this invention is comprised of amphoteric, nonionic, and cationic surfactants. Each of these surfactants are typically present in the antimicrobial system of this invention ranging from 0.1 to 15, preferably 0.1 to 8, most preferably 0.2 to 5% by weight.

Examples of suitable amphoteric surfactants include those related or derived from betaines such as amine betaines and amido betaines. Also useful amphoteric surfactants include glycinate and/or imidazole derivatives such as coco-imidazoline mono-carboxylate and/or dicarboxylate. Preferred amphoteric sufactants for use with this invention include hydroxysultaine, cocamidropropyl betaine, and sodium laurimino-dipropionate, and disodium lauroamphodiacetate.

Nonionic surfactants are neutral molecules without any charge, and these compounds are very mild with poor foaming properties. Non-ionic compounds diminish surface tension and dissolve in water quite easily, but not in same way as common salt. They are equally soluble in oil, which is important in producing emulsions. In the presence of water, they do not form simple solutions, they form complexes known as hydrates. Applications for nonionics include solubilization and for cationics, conditioning. Examples : Alkyl phenol ethoxylates, fatty acid dialkanolmides, fatty acid monoalkanolamides, fatty acid ethoxylates, fatty alcohol ethoxylates, fatty amine ethoxylates, substituted phenol ethoxylates, vegetable oil ethoxylates, polyalkylglycosides, sucrose esters and glyceryl laurate.

Generally, preferred nonionic surfactants include condensation products of one or more alkylene oxide groups with an organic hydrophobic compound, such as an aliphatic or alkyl aromatic compound. Exemplary nonionic surfactants based upon polyethoxylated, polyproproxylated, or polyglyceroxylated alcohols, alkylphenols, or fatty acids.

Further specific examples of nonionic surfactants include, for example, alkyl phenoxypolyethoxy ethanols having alkyl groups from about 7 to 18 carbon atoms and from about 6 to about 60 oxyethylene units such as, for example, heptyl phenoxypolyethoxyethanols, ethylene oxide derivatives of long chained carboxylic acids such as lauric acid, myristic acid, palmitic acid, oleic acid, and the like, or mixtures of acids such as those found in tall oil containing from about 6 to 60 oxyethylene units; ethylene oxide condensates of long-chained alcohols such as octyl, decyl, lauryl, or cetyl alcohols containing from 6 to 60 oxyethylene units; ethylene oxide condensates of long-chain or branched chain amines such as dodecyl amine, hexadecyl amine, and octadecyl amine, containing from about 6 to 60 oxyetheylene units; and block copolymers of ethylene oxide sections combined with one of more hydrophobic propylene oxide sections.

Examples of cationic surfactants include, for example, lauryl pyridinium chloride, cetyldimethyl amine acetate, and alkyldimethylbenzylammonium chloride, in which the alkyl group has from 8 to 18 carbon atoms.

Other useful cationic surfactants include aliphatic fatty amines and their derivatives, homologues of aromatic amines having fatty chains - dodecylaniline, fatty amides derived from aliphatic diamines, fatty amides derived from disubstituted amines, quaternary ammonium compounds, amides derived from aminoalcohols and their quaternary ammonium derivatives, quaternary ammonium bases derived from fatty amides of disubstituted diamines, quaternary ammonium bases of the benzimidazolines, basic compounds of pyridinium and its derivatives, quaternary ammonium compound of betaine, dimethylphenylbenzyl ammonium chloride, urethanes or basic salts of ethylene diamine, polyethylene diamines and their quaternary ammonium compounds.

A particularly useful mixture of surfactants comprise from 0.1 to 10% active weight % of cocamidopropyl hydroxysultaine (amphoteric surfactant), from 0.1 to 10% active weight % of polyalkylglycoside (preferably Plantaren 2000 from Henkel), nonionic surfactant, and from 0.1 to 10 by active weight % of PPG-40 diethylmonium chloride (Preferably Emcol CC-42 from Witco Chem. Co.), cationic surfactant.

The mixture of amphoteric, nonionic, and cationic surfactants of this invention have been shown to be compatible with high alcohol and low water systems, thereby resulting in a stable formulation.

The alcohol used with the composition of this invention is typically present in an amount ranging from 20 to 80%, preferably 40 to 80%, most preferably 60 to 70% by volume of the composition. The alcohols useful in the present invention include ethyl alcohol, iso-propyl alcohol, n-propyl alcohol and combinations thereof. Ethyl alcohol may be used as the only alcohol or the alcohol may be a mixture from 10 to 70% by volume ethyl alcohol, from 10 to 70% by volume iso-propyl alcohol, and from 10 to 70 % by volume n-propyl alcohol.

Other materials may be added to the compositions of this invention to improve such characteristics as skin conditioning and moisturization of the compositions. Thus, humectants such as glycerin, anti-inflammatory/anti-irritants such as isolene (C₁₂-C₁₈ diglycerides), anchoring agents, conditioners such as phenylethyl dimethicone (Silsoft PEDM from Witco OSi), silicone quaternary compound (e.g., Lambent Quat AD from Lambent Technologies, A Petroferm Company), cetrimonuim chloride, and glyceryl laurate. Glyceryl laurate (a non-ionic surfactant) in addition to contributing to conditioning and penetration of the antimicrobial compositions disclosed herein, also acts as a foam booster.

Typically, these additional agents may be present in the compositions of this invention according to the following amounts: glycerin from 0.1 to 40% by weight, phenylethyl dimethicone from 0.01 to 0.5% by weight, silicone quaternium 8 from 0.1 to 5% by weight, cetrimonium chloride from 0.1 to 5% by weight and glyceryl laurate from 0.5% to 10% by weight of the composition of this invention.

The antimicrobial compositions of the present invention are effective in controlling microorganisms when an effective amount of the composition is topically applied to a substrate or location, such as the hands, acne sites, patient prepping sites, or injection site for catheters, etc. The amount applied to be effective depends upon such environmental factors as the length of application, the amount of contact of the antimicrobial composition and the substrate, the condition of substrate (e.g., normal or dry skin) as well temperature and evaporation rates. Those with skill in the art will readily be able to determine the effective level necessary to control the microorganisms. Typically, from 0.5 to 10 milliliters, preferably from 1.0 to 9, and most preferably from 2.5 to 5 milliliters of the antimicrobial composition is applied. This amount of the antimicrobial composition if found to be effective, to provide a log₁₀ reduction of >1.0 or more in the microbe population. Also, the amount is enough to exhibit residual and cumulative antimicrobial effects on resident skin flora.

The present invention can also be prepared as an emulsion using techniques well known in the art, see for example US Patent No. 5,308,890. The active ingredients, excipients, etc., may be emulsified with amphoteric, cationic, and nonionic surfactants in the amounts previously noted.

### EXAMPLES

The following examples are illustrative of the present invention and are not intended to limit the invention to the following compositions. Unless noted to the contrary, all percentages presented in this application are understood to be weight percent.

The following formulations were applied to the skin following a modified surgical scrub procedure identified and described as Scrub Procedure One. The formulations were subsequently tested by the methods hereinafter described for antimicrobial effectiveness.

### Scrub Procedure One [Dry application, rub, dry application, rub, wet, lather, rinse]

Step 1.1: Volunteers' fingernails are checked to determined if they are <1.0 mm free edge. If not, they are clipped. Remove all jewelry from hands and arms.
Step 1.2: Subjects wet their hands including two-thirds of forearms under running tap water 40 ± 2°C for 30 seconds. Clean under fingernails and around the cuticle area with a nail cleaner. Rinse fingernails, cuticles, and hands.
Step 1.3: Subjects dry hands thoroughly with paper towels.
Step 1.4: Dispense into the subject's hands 5 ml of the assigned test article. Subjects are to distribute the material over all surfaces of the hands and lower two-thirds of the forearms taking care not to lose the substance.
Step 1.5: The material is vigorously rubbed over the hands and lower two-thirds of the forearms. Particular attention is paid to the nails, cuticles and interdigital spaces. Note: This step is performed over a period of approximately one-minute.
Step 1.6: Dispense a second 5 ml aliquot of the test article in the subject's cupped hands.
   Subjects are to distribute it over all the surfaces of the hands and lower one-third of the forearm, taking care not to lose the substance.
Step 1.7: Repeat the treatment procedure described in step 1.5 except limit the scrub to the hands and lower one-third of the forearms. (An additional one minute of rubbing time)
Step 1.8: Subjects wet hands under tap by passing hands one or two times through water.
Step 1.9: The test article is vigorously rubbed over the hands and lower one-third of the forearms paying particular attention to the finger nail region. Note: this lathering step is performed over a period of one minute.
Step 1.10: Rinse each hand and forearm separately for one minute per hand and shake to remove excess water.
Step 1.11: Proceed with antimicrobial effectiveness testing.

### Total Rubbing/Lathering time: 3 minutes.

The following compositions were used in the formulations hereinafter described:
AMP 95 is a mixture of 2-amino-2-methyl-1-propanol, 2-(methylamino)-2-methyl-1-propanol and water in a ratio of from about 90:5:5, commercially available from Angus Chemical Company.
ACRITAMER® 505E. a polyvinyl carboxy polymer crosslinked with ethers of pentaerythritol, R.I.T.A available from Crystal Lake, IL.
AMPHOTERGE K-2, coco imidazoline dicarboxylate, available from Lonza.
ESS 9090IC is a fragrance, available from Givuan-Roure Corporation.
CERAPHYL 28 is a mixture of cetyl alcohol and cetyl lactate, a waxy solid commercially available for ISP Van Dyk Inc.
CERAPHYL 41 is a mixture of C₁₂ - C₁₅ alcohol lactates, available from ISP Van Dyk Inc.
CETIOL HE- PEG-7 glyceryl cocoate, from Henkel.
COSMOCIL CQ is polyhexamethylene biguanide, available from Zeneca.
DISODIUM EDTA, U.S.P., available from Dow Chemical as Versene NA.
DOW CORNING® 580 wax is a mixture of stearoxy trimethoxy silane and stearyl alcohol.
DOWICIL 200, quaternium 15, Dow Chemical.
EMCOL CC42- PPG-40 dimonium chloride, or quaternium 21, available from Witco Corp.
GERMABEN II is a mixture comprised of diazolindinyl urea (about 30%); methyl paraben (about 11%); propyl paraben (about 3%) and propylene glycol (about 56%), available from Sutton Laboratories.
GERMALL PLUS is a mixture of diazolidinyl urea (about 99%), 3-Iodo-propynylbutylcarbamate available from Sutton Laboratories.
INCROMECTANT LAMEA- a mixture of acetamide monoethanolamine, and lactamide monoethanolamine (Croda)
LEXOREZ 100 is a saturated crosslinked hydroxy functional; polyester, comprised of glycerin, diethylene glycol, adipate crosslinked polymer, which is a viscous, hydrophobic liquid at room temperature and is dispersible in many lipids and emollients.
LEXQUAT AMG-IS, isostearamidopropyl PG dimonium chloride (Inolex Chemical Company)
MACKAM CBS-50G, cocamidopropyl hydroxysultaine, 50% (McIntyre)
MEARLMAID OL contains isopropyl alcohol, guanine, and Polysorbate 80 (Engelhard).
MIRATAINE CB - cocamidopropyl betaine (Rhone-Poulenc)
NATROSOL 250 HHR - hydroxyethylcellulose (Aqualon, Div. Of Hercules).
NISIN, a 34 amino acid polypeptide, sold as Ambicin by Applied Microbiology, Inc.
ORANGE ZEST B FRAGRANCE, a blend of oily volatile compounds, sold by Firmenich, Inc.
PEG-7'Glyceryl Cocoate (see Cetiol HE)
PEO-1 - polyethylene glycol, 21,000 M.W. INCI: PEG-5M (R.I.T.A.)
PHOSPOLIPID CDM is cocophosphatidyl (PG)-dimonium chloride, a co-synthetic, phospholipid available from Mona Industries, Inc.
PHOSPHOLIPID GLA - borageamidopropyl phosphatidyl PG-dimonium chloride (Mona).
PHOSPOLIPID PTC is cocamidopropyl phosphatidyl PG-dimonium chloride, available form Mona Industries.
PLANTAREN 2000 is decyl polyglucose, available from Henkel/Cospha.
SILSOFT PEDM is phenylethyl dimenthicone, available from Witco Cooperation, Osi Specialties, Inc.
SEAFOAM 143.258/GGE, fragrance available from Firmenich, Inc.
TOCOPHEROL (dl-alpha-tocopherol), Vitamin E, available from Roche Vitamins and Fine Chemicals.
TRICLOSAN - 2, 4, 4'-trichloro-2-hydoxydiphenyl ether.
ULTREZ® 10 a carbomer polymer, available from BF Goodrich, Cleveland Ohio, and disclosed in US patent 5, 004,598, the contents of which are incorporated by reference in its entirety.
VAROX 270 lauramine oxide, 30% active of 70% C₁₂, available from Witco.

### EXAMPLE 1

This is a comparative example to demonstrate the shortcoming of using antimicrobial systems containing anionic surfactants (i.e., ammonium laureth sulfate) in terms forming formulations of long-lasting (i.e., 6 hours) antimicrobial effectiveness.

### Formulation 1-1:

Ethanol (53.1%, 62% V/V), D.I. Water (23.2%), Zinc Oxide (0.5%), Glycerin (5%), PEG-7 Glyceryl Cocoate (1.0%), Lexorez 100 (1.5%), Silsoft A-843 (0.5%), Lexquat AMG-IS (1%), Incromectant LAMEA (1.5%), Tocopherol (0.2%), Ceraphyl 41 (0.5%), Natrosol 250 HHR (1%), PEO-1 (0.1%), Seafoam fragrance (0.15%),Plantaren 2000 (4%), Ammonium Laureth Sulfate (5%), EtOH (53.1% W/W), Phenoxyethanol (0.55%), Benzalkonium Chloride [50% solution] (0.22%), Germall Plus (0.11%), Germaben II (0.11%), Phospholipid CDM (0.5%), Phospholipid GLA (0.1%).

### Formulation 1-2 (w/triclosan):

Ethanol (46.2%, 55% V/V),D.I. Water (29.24%), Zinc Oxide (0.5%), Glycerin (5%), Cetiol HE (1.0%), Lexorez 100 (1.5%), Silsoft A-843 (0.5%), Lexquat AMG-IS (1%), Incromectant LAMEA (1.5%), Tocopherol (0.2%), Ceraphyl 41 (0.5%), Natrosol 250 HHR (1%), PEO-1 (0.1%), Seafoam fragrance (0.15%),Plantaren 2000 (4%), Ammonium Laureth Sulfate (5%), EtOH (46.2% W/W), Phenoxyethanol (0.55%), Benzalkonium Chloride [50% solution] (0.22%), Germall Plus (0.11%), Germaben II (0.11%), Phospholipid CDM (0.5%), Phospholipid GLA (0.1%), Triclosan (1.0%).

### Formulation 1-3 (w/ Australian Tea Tree Oil):

Ethanol (53.2%, 62% V/V), D.I. Water (22.4%), Zinc Oxide (0.5%), Glycerin (5%), Cetiol HE (1.0%), Lexorez 100 (1.5%), Silsoft A-843 (0.5%), Lexquat AMG-IS (1%), Incromectant LAMEA (1.5%), Tocopherol (0.2%), Ceraphyl 41 (0.5%), Natrosol 250 HHR (1%), PEO-1 (0.1%), Seafoam fragrance (0.15%),Plantaren 2000 (4%), Ammonium Laureth Sulfate (5%), EtOH (53.1% W/W), Phenoxyethanol (0.55%), Benzalkonium Chloride [50% solution] (0.22%), Germall Plus (0.11%), Germaben II (0.11%), Phospholipid CDM (0.5%), Phospholipid GLA (0.1%), Australian Tea Tree Oil (1.0%).

The pH of the preceding formulations was adjusted with phosphoric acid to 6.5.

The results of antimicrobial effectiveness of the foregoing formulations are summarized in TABLE 1 in terms of the cumulative and persistent activity for 1, 2, and 5 days at 0 and 6 hours as measured by the log₁₀ reductions. Briefly, the log₁₀ reduction test method is conducted on subjects selected from a group of volunteers who have refrained from using any antimicrobials for at least two weeks prior to initiation of the test. Sufficient number of subjects are selected from this group on the basis of high initial bacteria count, 1 X 10⁵ per hand as determined by baseline measurements of the bacteria on their hands.

The selected subjects perform a simulated surgical handwash under the supervision of an individual competent in aseptic technique. One hand is sampled after the surgical handwash and the other hand after 6 hours. The difference between the base line and the recovered organisms after surgical hand wash gives the antimicrobial effectiveness of test formulations.

Those with skill in the art will appreciate that the compositions with higher log₁₀ reduction value indicates improved efficacy. The log₁₀ reduction is the difference in the initial bacterial counts and the count recovered after each treatment.

**TABLE 1**

| | | | | | | |
|---|---|---|---|---|---|---|
| Time | 0 hr | 6 hr | 0 hr | 6 hr | 0 hr | 6 hr |
| Formulation | 1-1 | 1-1 | 1-2 | 1-2 | 1-3 | 1-3 |
| Day 1 | 0.58 | -0.1 | 0.68 | -0.15 | 0.59 | 0.38 |
| Day 2 | 0.52 | -0.25 | 0.89 | -0.0008 | 1.01 | 1.05 |
| Day 5 | 1.13 | 0.30 | 1.02 | 0.56 | 1.60 | 1.79 |

Cumulative activity of the tested formulations was evaluated by comparing the log₁₀ reductions achieved at 0 hours on day 1 to the log₁₀ reductions achieved at 0 hours on day 2 and 5. The paired "t- test" results of these comparisons indicated significantly more antimicrobial activity on days 2 and 5 at 0 hours compared to day 1 at 0 hours for Formulation 1-3. Significantly more antimicrobial activity was indicated on day 5 but not day 2 at 0 hours compared to day 1 at 0 hours for Formulation 1-1 and Formulation 1-2. Those with skill in the art will appreciate that the "t- test" is a statistical method used to compare the test material from the control to establish the significance at 0.05 level of significance. This compares the differences between means of the two distributions divided by the flux about those means. This then is the "t" value for that difference. The larger the "t" value the greater the probability that the two means are different because they come from distinct rather than just random sampling chance.

Mathematically speaking "t" values become large as: 1) the difference between the two means gets larger; and 2) the flux about the mean (standard deviations) get smaller.

However, the low log₁₀ reductions and weak persistent activity of all tested formulations, which fall well short of FDA requirements for a surgical scrub, are believed to be attributed to inactivation of the antimicrobial compositions, i.e., by the high-foaming anionic-based surfactant system, i.e., the ammonium laureth sulfate. Thus, the foregoing formulations do not provide an adequate solution to the problem of providing long-lasting antimicrobial effectiveness.

### EXAMPLE 2

In view of the results of Example 1, the following formulations free of anionic surfactants were screened for in vivo antimicrobial efficacy both at 0 Time and 6 Hours (to test for cumulative or residual effects).

### Formulation 2-1:

EtOH (61.8% W/W or 70% V/V), D.I. Water (18.51%), Mirataine CB (6.0 %), Glycerin (2.5%), Amphoterge K-2 (2%), HCl 1 N (1.8%), Cetiol HE (1.0%), Zinc Oxide (0.5%), Lexorez 100 (0.75%), Silsoft A-843 (0.5%), Ceraphyl 41 (0.5%), Natrosol 250 HHR (0.8%), PEO-1 (0.1%), Seafoam fragrance (0.15%),), Varox 270 ( 0.5%), , Phenoxyethanol (0.55%), Benzalkonium Chloride [50% solution] (0.22%), Germall Plus (0.11%), Germaben II (0.11%), Phospholipid CDM (0.5%), Phospholipid GLA (0.1%), Propylene Glycol (0.5%), and Propylene carbonate (0.5%).

### Formulation 2-2:

EtOH (52.9% W/W or 60 v/v), Isopropyl alcohol (4.38 W/W or 5 v/v), n-Propyl alcohol (4. 49 w/w or 5 v/v), D.I. Water (18.51%), Mirataine CB (6.0 %), Glycerin (2.5%), Amphoterge K-2 (2%), HCl 1 N (1.8%), Cetiol HE (1.0%), Zinc Oxide (0.5%), Lexorez 100 (0.75%), Silsoft A-843 (0.5%), Tocopherol (0.2%), Ceraphyl 41 (0.5%), Natrosol 250 HHR (0.8%), PEO-1 (0.1%), Seafoam fragrance (0.15%),), Varox 270 (0.5%), Phenoxyethanol (0.55%), Benzalkonium Chloride [50% solution] (0.22%), Germall Plus (0.11%), Germaben II (0.11%), Phospholipid CDM (0.5%), Phospholipid GLA (0.1%), Propylene Glycol (0.5%), and Propylene carbonate (0.5%).

### Formulation 2-3 (w/Triclosan):

EtOH (48.42% W/W or 55 v/v), Isopropyl alcohol (8.76 W/W or 10 v/v), n-Propyl alcohol (4.48 w/w or 5 v/v), D.I. Water (18.51%), Mirataine CB (6.0 %), Glycerin (2.5%), Amphoterge K-2 (2%), HCl 1 N (1.8%), Cetiol HE (1.0%), Zinc Oxide (0.5%), Lexorez 100 (0.75%), Silsoft A-843 (0.5%), Tocopherol (0.2%), Ceraphyl 41 (0.5%), Natrosol 250 HHR (0.8%), PEO-1 (0.1%), Seafoam fragrance (0.15%),), Varox 270 (0.5%), , Phenoxyethanol (0.55%), Benzalkonium Chloride [50% solution] (0.22%), Germall Plus (0.11%), Germaben II (0.11%), Phospholipid CDM (0.5%), Phospholipid GLA (0.1%), Propylene Glycol (0.5%), Propylene carbonate (0.5%), and Triclosan (1.0 %) .

### Formulation 2-4 (w/Triclosan & Australian Tee Tree Oil) :

EtOH (48.42% W/W or 55 v/v), Isopropyl alcohol (8.76 W/W or 10 v/v), n-Propyl alcohol (4.48 w/w or 5 v/v), D.I. Water (18.51%), Mirataine CB (6.0 %), Glycerin (2.5%), Amphoterge K-2 (2%), HCl 1 N (1.8%), Cetiol HE (1.0%), Zinc Oxide (0.5%), Lexorez 100 (0.75%), Silsoft A-843 (0.5%), Tocopherol (0.2%), Natrosol 250 HHR ( 0.8%), PEO-1 (0.1%), Orange Zest fragrance (0.2%),), Varox 270 (0.5%), Phenoxyethanol (0.55%), Benzalkonium Chloride [50% solution] (0.22%), Germall Plus (0.11%), Germaben II (0.11%), Phospholipid CDM (0.5%), Phospholipid GLA (0.1%), Propylene Glycol (0.5%), Propylene carbonate (0.5%), Australian tea tree oil (1.0%), and triclosan (1.0 %).

The results of the antimicrobial efficiency for the foregoing formulations are summarized in TABLE 2.

**TABLE 2**

| **Formulation** | **Sampling Period** | **Mean LOG**_{**10**} **Reduction** |
|---|---|---|
| 2-1 | 0 Hour, Day 1 | 0.48 |
| 2-2 | | 0.39 |
| 2-3 | | 0.25 |
| 2-4 | | 0.28 |

Referring to TABLE 2, it was surprising that none of these formulations met the FDA requirement for 1 Log₁₀ reduction even for zero time on day 1. Although not reported in Table 2, the formulations containing Triclosan, i.e., Formulations 2-3 and 2-4, showed greater cumulative activity than the others at days 2 and 5. Thus, simple elimination of anionic surfactants did not appear the only factor affecting antimicrobial activity. Since poor 0 hour results were achieved for formulations 2-1 to 2-4, the 6 hour results are not reported.

### EXAMPLE 3

In view of the results of Examples 1 and 2 and in order to more quickly receive and evaluate results, it was decided to perform in vitro evaluations of various combinations of surfactants and antimicrobials.

The in vitro time kill study was conducted with 9 microorganisms by evaluating the log₁₀ reductions of bacterial counts using 8 log₁₀ bacterial inoculation into each test product. All subsequent time-kill studies for the brushless scrub were conducted under this protocol. The microorganisms (ATCC and clinical isolates) tested are identified in the following tables by both the commonly used descriptive names of the microorganisms and by the ATCC identification numbers.

The previously referenced formulations, Formulations 2-1, 2-2, 2-3 and 2-4, as well as alcohol-containing Formulations 2-5 and 2-6 were evaluated by this time-kill method. Formulation 2-5 contained ethyl alcohol 49.1%, isopropyl alcohol 8.9%, n-propyl alcohol 4.5%, and water 37.5% based on W/W% and Formulation 2-6 contained simply 70% V/V% ethanol in water. TABLE 3 represents the results of the antimicrobial efficacy of the foregoing formulations in terms of log₁₀ and percentage bacterial kill.

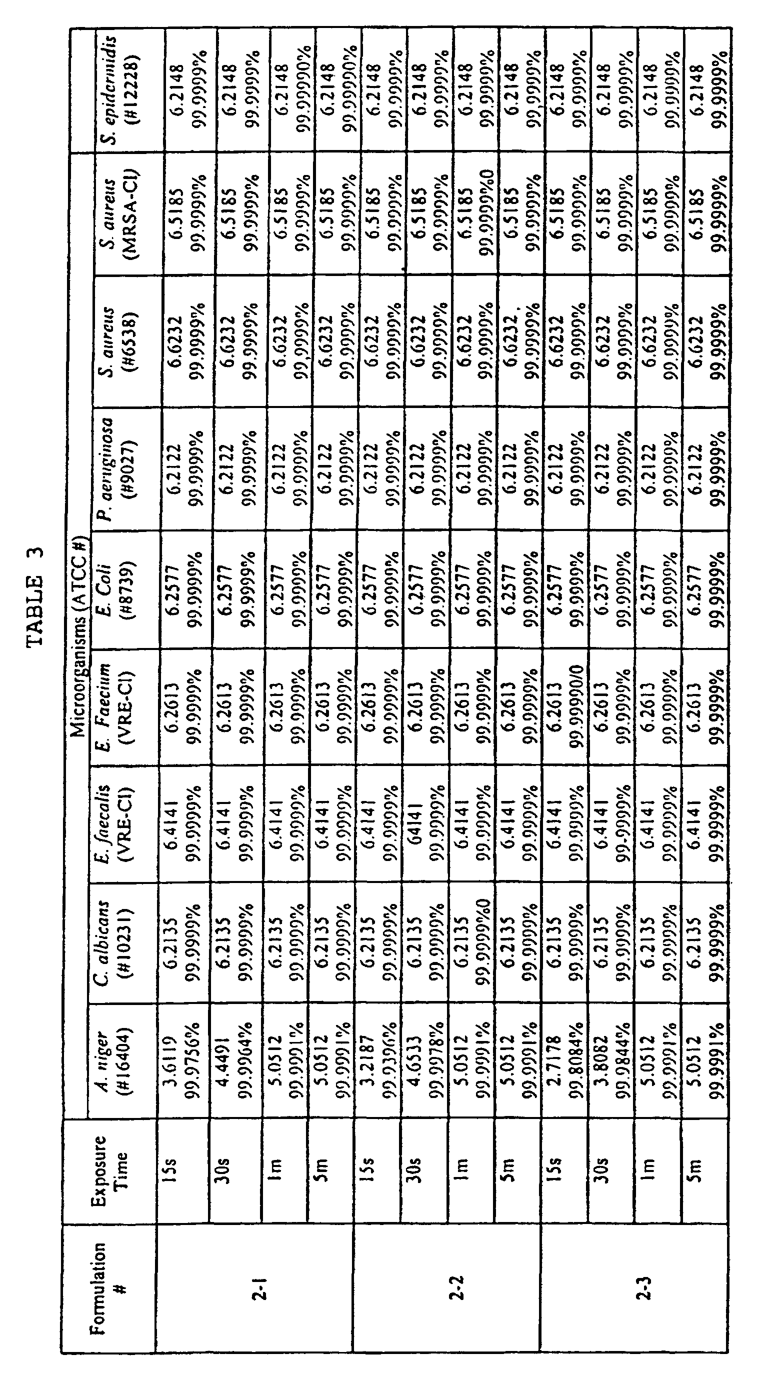

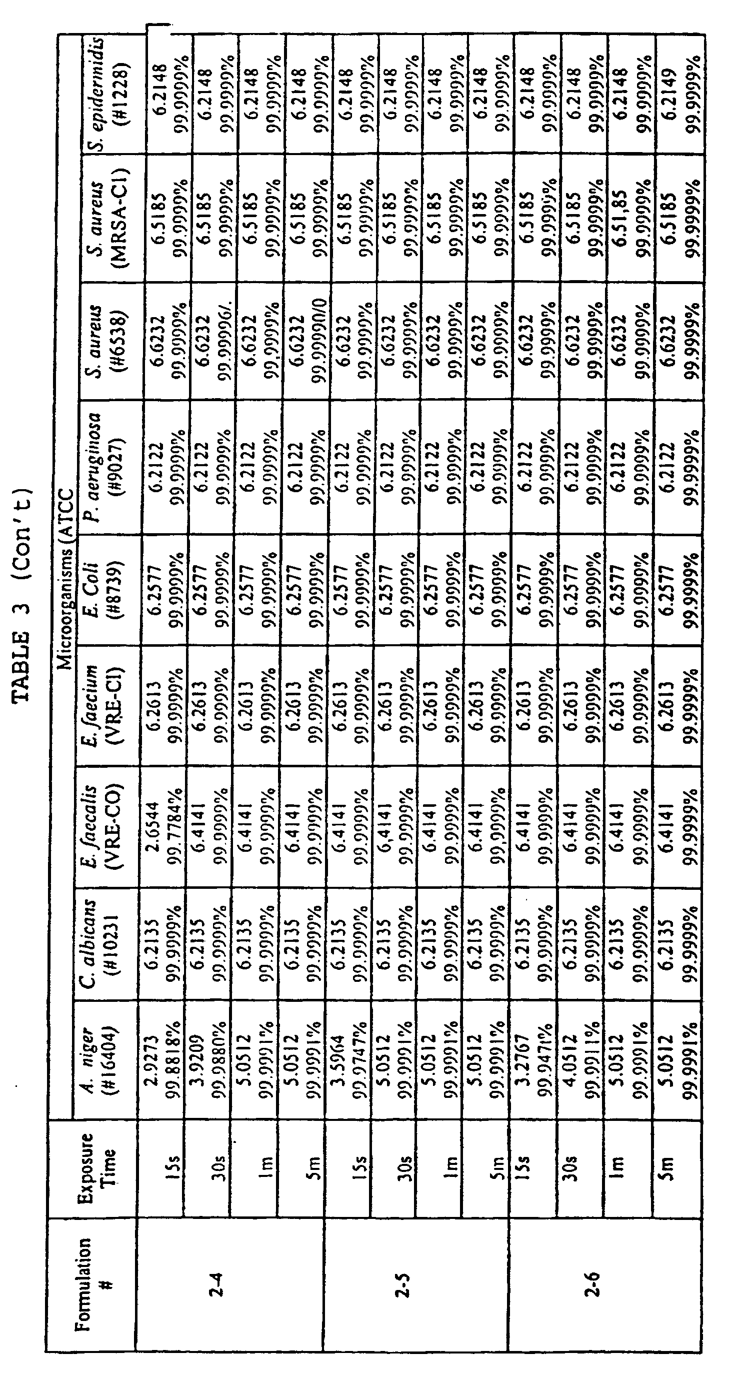

Referring to TABLE 3, it is clear that log₁₀ reductions at 15 seconds were from 5 to 6 across the board for these formulations, except in the case of the microorganism A. niger (ATCC #16404), in which the log₁₀ reduction which was typically 3 for all formulations measured at 15 seconds. The kill was overwhelming in these formulations due to the presence of alcohol (typically 6 log in 15 s), and differences between them were lost.

### EXAMPLE 4

Based on the results of Example 3 and in an attempt to isolate the effectiveness of antimicrobial systems without alcohol the following in vitro samples submitted were in an aqueous base only, and contained no alcohol. In the Formulation 4 series, a common antimicrobial base was combined with three surfactant variations. The base contained Benzalkonium Chloride (0.09% active), Benzethonium Chloride (0.09% active), Phenoxyethanol (0.5%), Phospholipid CDM (1.0%), Propylene Glycol (3.33%), Glycerin (1.67%), and water. Formulation 4-1 contained Ammonium Laureth Sulfate(2%), an anionic surfactant, and 0.1% Cetylpyridinium Chloride additionally. Formulation 4-2 contained the base plus Cocamidopropyl Hydroxysultaine (Mackam CBS 50G, 2.0%) and Cetylpyridinium Chloride (0.25%). Formulation 4-3 contained the base plus PPG-40 Diethylmonium Chloride (Emcol CC42, 2.0%) and Cetylpyridinium Chloride (0.5%).

It was noticed that version Formulation 4-1 formed a precipitate. It is likely due to the incompatibility of the cationic quaternary compounds (Benzalkonium Chloride, Benzethonium Chloride, Cetylpyridinium Chloride) with the anionic Ammonium Laureth Sulfate. Formulations 4-2 and 4-3 remained clear. Results of the antimicrobial efficiency of the foregoing formulations are presented in TABLE 4.

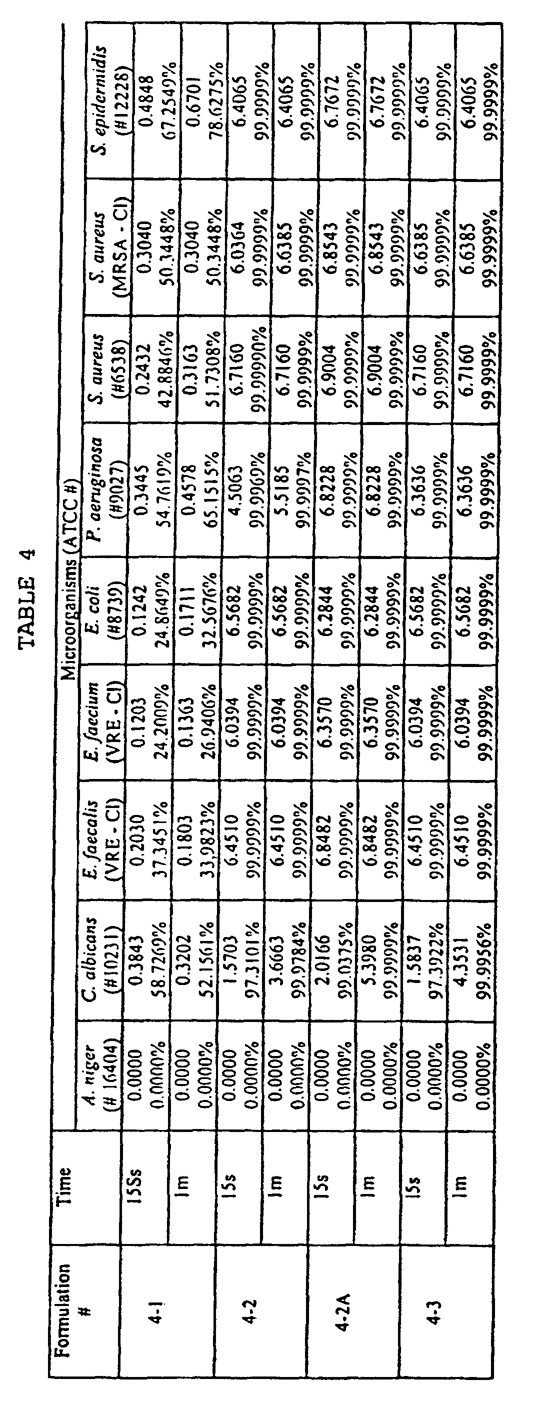

Referring to TABLE 4, it is clear that Formulation 4-1 exhibited very poor antimicrobial activity in the time-kill studies compared to Formulations 4-2 and 4-3, confirming the likely inactivation of the cationic antimicrobials.

These results suggest the complete compatibility and possible enhancement of the antimicrobial system by Cocamidopropyl Hydroxysultaine and PPG-40 Dimonium Chloride at 2.0% levels.

To improve foaming and mildness, a third surfactant was tested in Formulation 4-2A, Plantaren 2000 (polyalkylglycoside), substituting this surfactant for Cocamidopropyl Hydroxysultaine in Formulation 4-2 . The results showed excellent activity for this formula as well, with no apparent suppression of the antimicrobials. (See TABLE 4).

At this point we had three viable surfactants compatible with our antimicrobial system, Plantaren 2000 (a non-ionic), Cocamidoprcpyl Hydroxysultaine (an amphoteric), and PPG-40 Dimonium Chloride (a cationic). The combination of the three was found to give good foaming and lather.

### EXAMPLE 5

This example investigates the effect of pH on the antimicrobial systems of this invention. Up to this point, all formulas tested were in the pH 6-7 range. A new formulation, Formulation 5-1 was made which contained the previously mentioned antimicrobial system, of Formulation 4-2 plus Nisin (0.1%), Disodium EDTA (0.1%), a surfactant system consisting of Plantaren 2000 (nonionic) and Mackam CBS-50G (amphoteric), and pH adjuster Glycolic Acid (0.19% of a 70% solution). The pH of this batch was 3.5.

The time-kill efficacy results for this formula showed weak activity. The log₁₀ reductions were less than 1 for many of the microorganisms at the 15 second interval. This implied that there was no benefit and probably deleterious effects on efficacy from low pH with this antimicrobial system. We also attempted a high pH formula, Formulation 5-2, contained the following based on weight %: Deionized water 97%; PEG-4 cellulose 0.5%; benzalkonium chloride (50%) 0.18%; Benzethonium chloride 0.09%; Cocamidopropyl Hydroxysultaine 2.0%; with the pH adjusted to 8.3 with 10% NaOH. The efficacy at this pH was also weaker than at the apparent optimum pH of approximately 7. As we had seen excellent efficacy results from formulas of pH approximately 7, we decided to make that the target pH. In addition, this is the pH where this particular system with these surfactants and antimicrobials is most stable, requiring no adjustment. Thus, best antimicrobial performance would be expected in pH ranges around 7, most likely from about 5.5 to about 8.

### EXAMPLE 6

Now that we had identified an excellent surfactant system by in vitro testing (the Plantaren 2000, Cocamidopropyl Hydroxysultaine, and PPG-40 Dimonium Chloride combination), it was decided to test some variations on this theme, using an in vivo scrub study with two more formulations, Formulations 6-1 and 6-2. Formulation 6-1 contained EtOH (26.5% W/W or 30% V/V), n-Propyl Alcohol (25.1% W/W or 28% V/V), Triclosan (1.0%), D.I. Water (27.67%), Opacifier-295 (Morton), Hydroxypropylcellulose (1.0%), Plantaren 2000 (3.0), Cocamidopropyl Hydroxysultaine -Mackam CBS50G (2.0%), PPG-40 Diethylmonium Chloride-Emcol CC42 (1.0%), Benzalkonium Chloride [50% solution] (0.18%), Benzethonium Chloride (0.09%) ,Phenoxyethanol (0.5%), Phospholipid CDM (0.5%), Phospholipid GLA (0.5%), Cetrimonium Chloride (0.86% of 29% sol.), Dowicil 200 (0.1%), Cetylpyridinium Chloride (0.25%), Glycerin (5%), Propylene Glycol (0.5%), fragrance (0.15%). Formulation 6-2 contained the same composition as Formulation 6-1 except that Formulation 6-2 is a high glycerin formula (25%), high-alcohol (65% V/V-active levels), and low water formula and whereas Formulation 6-1 is a mixed alcohol formula (total of 58% V/V, or an inactive level), and the glycerin has been reduced to 5%.

Also tested in this study was Prevacare Antimicrobial Hand Gel (Lot No. P8-006), Healthpoint's Triseptin Surgical Scrub, and pure ethanol at 70% V/V. Only 1st day results at 0 time were evaluated and are shown in Table 6.

**TABLE 6**

| **Formulation** | **Sampling Period** | **Mean LOG**_{**10**} **Reduction** |
|---|---|---|
| 6-1 | 0 Hour, Day 1 | 1.18 |
| 6-2 | | 1.24 |
| Antimicrobial Hand Gel (60% Ethanol) | | 1.40 |
| Surgical Scrub (70% Ethanol) | | 1.52 |
| Ethanol 70% | | 0.95 |

The results of Table 6 indicate that all the formulations met the FDA surgical scrub requirement for 1-log₁₀ reduction in bacteria at zero time with the exception of Ethanol 70%.

These were the first formulas tested in vivo which met the FDA requirements for rapid kill. This confirmed the compatibility of this particular surfactant mixture with the antimicrobial mixture. In the presence of alcohol, this combination met the surgical scrub requirements when utilizing a double-dry application, lather, and rinse method.

Also, notable from these results is that the formula did not have statistically significant gains in activity from the presence of Triclosan, at least at 0 time. The cumulative efficacy effects of each formula are unknown from this study which only evaluated 0 time results.

### EXAMPLE 7

In an effort to further increase the antimicrobial efficacy of the formula and the moisturization, further adjustments to the formula were made.

The improved formula is as follows:
Formulation 7-1:

EtOH (62.25% W/W or 70% V/V), D.I. Water (12.74%), Glyceryl Laurate (1.0%), Isolene (1.0%), Silsoft PEDM (0.05%), Mearlmaid OL (0.1%), Hydroxypropylcellulose (1.0%), Plantaren 2000 (3.0), Cocamidopropyl Hydroxysultaine -Mackam CBS50G (2.0%), PPG-40 Diethylmonium Chloride-Emcol CC42 (1.0%), Benzalkonium Chloride [50% solution] (0.18%), Benzethonium Chloride (0.09%), Phenoxyethanol (0.5%), Phospholipid CDM (0.5%), Phospholipid GLA (0.5%), Cetrimonium Chloride (0.86% of 29% sol.), Dowicil 200 (0.25%), Cetylpyridinium Chloride (0.25%), Glycerin (5%), Propylene Glycol (5%) and fragrance (0.15%),

Silsoft PEDM and Isolene both contribute to appearance and feel. They are both partially soluble in a hydroalcoholic system forming droplets, which help the opacity and lotion-like appearance of the product. Glyceryl Laurate was added at 1.0% to enhance the foaming and trans-dermal penetration abilities of the formula. The Phospholipid CDM and Benzalkonium Chloride were also increased in this formula to enhance efficacy and moisturization. Isolene and Phospholipid CDM also have anti-irritant benefits to compensate for increases in the Benzalkonium Chloride levels. Log₁₀ reduction data is shown in Table 7 for Formulation 7-1 for both 0 hour and 6 hours.

**TABLE 7**

| **Formulation** | **Log**_{**10**} **at 0 hours** | **Log**_{**10**} **at 6 hours** |
|---|---|---|
| 7-1 | 1.41 | 0.84 |

Thus, the results of Table 7 show extremely improved log₁₀ reductions at both 0 hour and 6 hour compared to the measured properties of previously tested formulations that do not contain the claimed elements of this invention most notably the formulations of Table 1.

### EXAMPLE 8

Two more formulations (Formulations 8-1 and 8-2) were evaluated. Formulation 8-2's surfactant system consisted of only cationic and nonionic surfactants. Formulation 8-1 contained (based on W/W%): 8.15% deionized water; 62.00% Ethanol (200 proof); 5.00% Glycerin; 10.00% Propylene Glycol; 5.00% Cocamidopropyl hydroxy sultaine (50% Concentration) Mackam CBS-50G(amphoteric); 1.00% Phospholipid CDM; 0.50% Phospholipid GLA; 1.20% PPG-40 Diethylmonium Chloride (Emcol CC-42) (cationic); 0.80% Hydroxypropylcellulose HXF Grade; 1.00% Phenoxyethanol; 1.50% Glyceryl Laurate (non-ionic) Monomuls 90-L12; 1.70% Cetrimonium Chloride (29%-Varisoft 300); 0.20% benzalkonium chloride (50%); 0.10% Benzethonium Chloride; 0.50% Lambent Quat AD; 0.15% Fragrance (Seafoam GGE); 1.00% Cosmocil CQ (polyhexamethylene biguanide 0.2%); 0.05% Silsoft PEDM; 0.15% Mearlmaid OL. Formulation 8-2 contained (based on W/W%): 9.83% deionized water; 62.75% Ethanol (200 proof); 10.00% Propylene Glycol; 5.0% Glycerin; 1.5% Phospholipid CDM; 1.5% PPG-40 Diethylmonium Chloride (Emcol CC-42); 0.80% Hydroxypropylcellulose HXF Grade; 1.0% Phenoxyethanol; 2.5% Glyceryl Laurate; 2.5% Cetrimonium Chloride (29%-Varisoft 300); 0.2% Benzalkonium Chloride (50%); 0.1% Benzethonium Chloride; 0.5% Lambent Quat AD; 0.15% Fragrance (Seafoam GGE); 1.5% Cosmocil CQ; .0.07% Silsoft PEDM; 0.100% Mearlmaid OL. Surprisingly Formulation 8-2 exhibited excellent foaming properties similar to surfactant systems containing amphoteric, nonionic, and cationic surfactants. The surprising observation was that one skilled in the art would have expected worse foaming properties due to the higher relative proportion of cationic surfactants in the system. However, no appreciable foaming differences were observed in the increased amounts of nonionic/cationic surfactant system when compared to the amphoteric/nonionic/cationic surfactant system. In fact, these observations in foaming ability and its believed correlation to skin penetration is at least borne out in the excellent antimicrobial results shown in TABLE 8 for Formulation 8-1 and compared with commercially available 4% chlorhexidine gluconate and 70% ethyl alcohol based products.

**TABLE 8**

| | **Log**_{**10**} **at 0 hours** | **Log**_{**10**} **at 6 hours** |
|---|---|---|
| Formulation 8-1 | 1.8 | 1.6 |
| HIBILCLENS | 1.6 | 1.9 |
| TRISEPTIN | 1.7 | 1.8 |

Formulation 8-1 was evaluated following the aforementioned new brushless surgical handwashing procedure (3 minute procedure with out a brush) that was based on surgical science and compared with conventional procedure (6 minute scrub with a brush) using 4% chlorhexidine gluconate product. Also the results were compared with a 70% alcohol based product following a 3 minute surgical scrub procedure with out a brush. To our surprise Formulation 8-1 has shown slightly better results at 0 hour and comparable activity at 6 hours. The results clearly suggest that Formulation 8-1 has the right combination of antimicrobial ingredients at appropriate concentrations to exhibit immediate and residual antimicrobial activity against resident skin flora which is relatively hard to achieve. This level of efficacy is an important feature in brushless applications to eliminate abrasive surgical scrub procedures with brushes and to offer the same level of efficacy of bench mark products, particularly, in half time of the conventional surgical scrub procedures with a brush.

## Claims

1. An antimicrobial handwash composition comprising:
a) an alcohol;
b) an effective amount of a cationic quaternary ammonium compound, phenoxy ethanol, and optionally a biguanide compound; and
c) an effective amount of surfactant system which is a mixture of nonionic and cationic surfactants or is a mixture of nonionic, cationic and amphoteric surfactants,
wherein: alcohol is from 30 to 65 percent by weight; the phenoxy ethanol is from 0.1 to 5.0 percent by weight; the cationic quaternary ammonium compound is from 0.02 to 2.5 percent by weight; and the surfactant system is 0.1 to 15 percent by weight.

2. The composition of claim 1 wherein the alcohol is ethyl alcohol, isopropyl alcohol, n-propyl alcohol or a mixture thereof.

3. The composition of claim 1 or claim 2 wherein the cationic quaternary ammonium compound is benzalkonium chloride, benzethonium chloride, cetylpyridinium chloride, cetrimonium chloride or a mixture thereof.

4. The composition of any one of claims 1 to 3 wherein the surfactant system is a mixture of glyceryl laurate and PPG-40 diethylmonium chloride.

5. The composition of any one of claims 1 to 3 wherein the surfactant system is a mixture of cocamidopropyl hydroxysultaine, polyalkylglycoside, and PPG-40 diethylmonium chloride.

6. The composition of any one of claims 1 to 5 wherein the alcohol comprises from 50 to 65 weight percent of alcohol referenced in claim 2; the cationic quaternary ammonium compound comprises from 0.01 to 0.5 percent by weight of benzalkonium chloride and from 0.01 to 0.5 percent by weight of benzethonium chloride; the surfactant system comprises from 0.1 to 8.0 weight percent of glyceryl laurate and from 0.2 to 5.0 weight percent of PPG-40 diethylmonium chloride.

7. The composition of any one of claims 1 to 5 wherein the alcohol comprises from 50 to 65 weight percent of alcohol wherein the alcohol is ethyl alcohol, isopropyl alcohol, n-propyl alcohol or a mixture thereof; the cationic quaternary ammonium compound comprises from 0.01 to 0.5 percent by weight of benzalkonium chloride and from 0.01 to 0.5 percent by weight of benzethonium choride; the surfactant system comprises from 0.1 to 8.0 weight percent of cocamidopropyl hydroxysultaine (50% concentration), from 0.2 to 5.0 weight percent of polyalkylglycoside, optionally glyceryl laurate from 0.1 to 8.0 weight percent, and from 0.2 to 5.0 weight percent of PPG-40 diethylmonium chloride.

8. The composition of any one of claims 1 to 7 wherein the composition contains an effective amount of a biguanide.

9. The composition of claim 8, wherein the biguanide is chlorhexidine or one of its derivatives, such as chlorhexidine gluconate, chlorhexidine gigluconate, chlorhexidine diacetate, chlorhexidine dihydrochloride and polyhexamethylene biguanide.

10. The composition of claim 8 wherein the biguanide is polyhexamethylene biguanide, chlorhexidine gluconate, or a mixture thereof.

11. The composition of any one of claims 8 to 10 wherein the biguanide is present in an amount from 0.01 to 5.0 weight percent.

12. The composition of any one of claims 1 to 11 wherein the composition contains an effective amount of skin conditioning system.

13. The composition of claim 12, wherein the skin conditioning system is comprised of propylene glycol, glycerin, phenylethyl dimethicone and a silicone quaternary compound.

14. The composition of claim 13, wherein the propylene glycol is present in an amount from 1.0 to 20 weight percent; glycerin in an amount from 1.0 to 40 weight percent; phenyl ethyl dimethicone in an amount from 0.01 to 0.2 weight percent; and silicone quaternary compound in an amount from 0.1 to 1.0 weight percent.

15. The composition of any one of claims 1 to 14 for use in a method of disinfecting a substrate such as the skin.

## Patentansprüche

1. Antimikrobielle Handwaschzusammensetzung, umfassend:
a) einen Alkohol;
b) eine effektive Menge einer kationischen quatärneren Ammoniumverbindung, Phenoxyethanol und gegebenenfalls einer Biguanidverbindung; und
c) eine effektive Menge eines oberflächenaktiven Systems, das ein Gemisch von nichtionischen und kationischen oberflächenaktiven Mitteln ist, oder ein Gemisch von nichtionischen, kationischen und amphoteren oberflächenaktiven Mitteln,
wobei: Alkohol in von 30 bis 65 Gewichtsprozent vorliegt; das Phenoxyethanol von 0,1 bis 5,0 Gewichtsprozent vorliegt, die kationische quatärnere Ammoniumverbindung von 0,02 bis 2,5 Gewichtsprozent vorliegt und das oberflächenaktive System von 0,1 bis 15 Gewichtsprozent vorliegt.

2. Zusammensetzung nach Anspruch 1, wobei der Alkohol Ethylalkohol, Isopropylalkohol, N-Propylalkohol oder ein Gemisch davon ist.

3. Zusammensetzung nach Anspruch 1 oder Anspruch 2, wobei die kationische quatärnere Ammonimumverbindung Benzalkoniumchlorid, Benzethoniumchlorid, Cetylpyridiniumchlorid, Cetrimoniumchlorid oder ein Gemisch davon ist.

4. Zusammensetzung nach einem der Ansprüche 1 bis 3, wobei das oberflächenaktive System ein Gemisch von Glyceryllaurat und PPG-40 Diethylmoniumchlorid ist.

5. Zusammensetzung nach einem der Ansprüche 1 bis 3, wobei das oberflächenaktive System ein Gemisch von Cocamidpropylhydroxysultanin, Polyalkylglycosid und PPG-40 Diethylmoniumchlorid ist.

6. Zusammensetzung nach einem der Ansprüche 1 bis 5, wobei der Alkohol von 50 bis 65 Gewichtsprozent von in Anspruch 2 angegebenem Alkohol aufweist; die kationische quatärnere Ammoniumverbindung von 0,01 bis 0,5 Gewichtsprozent Alkoniumchlorid und von 0,01 bis 0,5 Gewichtsprozent Benzethoniumchlorid aufweist; das oberflächenaktive System von 0,1 bis 8,0 Gewichtsprozent Glyceryllaurat und von 0,2 bis 5,0 Gewichtsprozent PPG-40 Diethylmoniumchlorid umfaßt.

7. Zusammensetzung nach einem der Ansprüche 1 bis 5, wobei der Alkohol von 50 bis 65 Gewichtsprozent an Alkohol umfaßt, wobei der Alkohol Ethylalkohol, Isopropylalkohol, N-Propylalkohol oder ein Gemisch davon ist; die kationische quatärnere Ammoniumverbindung umfaßt von 0,01 bis 0,5 Gewichtsprozent Benzalkoniumchlorid und von 0,01 bis 0,5 Gewichtsprozent Benzethoniumchlorid; das oberflächenaktive System umfaßt von 0,1 bis 8,0 Gewichtsprozent Cocamidopropylhydroxysultain (50 % Konzentration), von 0,2 bis 5,0 Gewichtsprozent Polyalkylglycosid, gegebenenfalls Glyceryllaureat von 0,1 bis 8,0 Gewichtsprozent, und von 0,2 bis 5,0 Gewichtsprozent PPG-40 Diethylmoniumchlorid.

8. Zusammensetzung nach einem der Ansprüche 1 bis 7, wobei die Zusammensetzung eine effektive Menge eines Biguanids enthält.

9. Zusammensetzung nach Anspruch 8, wobei das Biguanid Chlorhexidin oder eines seiner Derivate ist, wie z.B. Chlorhexidingluconat, Chlorhexidingigluconat, Chlorhexidindiacetat, Chlorhexidindihydrochlorid und Polyhexamethylenbiguanid.

10. Zusammensetzung nach Anspruch 8, wobei das Biguanid Polyhexamethylenbiguanid, Chlorhexidingluconat oder Gemische davon ist.

11. Zusammensetzung nach einem der Ansprüche 1 bis 10, wobei das Biguanid in einer Menge von 0,01 bis 5,0 Gewichtsprozent vorhanden ist.

12. Zusammensetzung nach einem der Ansprüche 1 bis 11, wobei die Zusammensetzung eine effektive Menge eines Haut-konditionierenden Systems enthält.

13. Zusammensetzung nach Anspruch 12, wobei das Haut-konditionierende System zusammengesetzt ist aus Polyethylenglycol, Glycerin, Phenylethyldimethicon und einer Silikon quatärneren Verbindung.

14. Zusammensetzung nach Anspruch 13, wobei das Propylenglycol in einer Menge von 1,0 bis 20 Gewichtsprozent vorhanden ist; Glycerin in einer Menge von 1,0 bis 40 Gewichtsprozent vorhanden ist; Phenylethyldimethicon in einer Menge von 0,01 bis 0,2 Gewichtsprozent vorhanden ist und Silikon quatärnere Verbindung in einer Menge von 0,1 bis 1,0 Gewichtsprozent vorhanden ist.

15. Zusammensetzung nach einem der Ansprüche 1 bis 14 zur Verwendung in einem Verfahren zur Desinfektion eines Substrats, wie z.B. der Haut.

## Revendications

1. Composition de lavage de mains antimicrobienne comprenant :
a) un alcool ;
b) une quantité efficace d'un composé d'ammonium quaternaire cationique, d'un phénoxyéthanol et facultativement d'un composé biguanide ; et
c) une quantité efficace de système tensioactif qui est un mélange de tensioactifs non ioniques et cationiques ou qui est un mélange de tensioactifs non ioniques, cationiques et amphotères,
dans laquelle : l'alcool représente de 30 à 65 % en poids, le phénoxyéthanol représente de 0,1 à 5,0 % en poids ; le composé d'ammonium quaternaire cationique représente de 0,02 à 2,5 % en poids ; et le système tensioactif représente de 0,1 à 15 % en poids.

2. Composition selon la revendication 1, dans laquelle l'alcool est l'alcool éthylique, l'alcool d'isopropyle, l'alcool de n-propyle ou un mélange de ceux-ci.

3. Composition selon la revendication 1 ou la revendication 2, dans laquelle le composé d'ammonium quaternaire cationique est de chlorure de benzalkonium, le chlorure de benzéthonium, le chlorure de cétylpyridinium, le chlorure de cétrimonium ou un mélange de ceux-ci.

4. Composition selon l'une quelconque des revendications 1 à 3, dans laquelle le système tensioactif est un mélange de laurate de glycéryle et de chlorure de PPG-40 diéthylmonium.

5. Composition selon l'une quelconque des revendications 1 à 3, dans laquelle le système tensioactif est un mélange de cocamidopropyl hydroxysultaine, de polyalkylglycoside et de chlorure de PPG-40 diéthylmonium.

6. Composition selon l'une quelconque des revendications 1 à 5, dans laquelle l'alcool comprend de 50 à 65 % en poids d'un alcool référencé dans la revendication 2 ; le composé d'ammonium quaternaire cationique comprend de 0,01 à 0,5 % en poids de chlorure de benzalkonium et de 0,01 à 0,5 % en poids de chlorure de benzéthonium ; le système tensioactif comprend de 0,1 à 8,0 % en poids de laurate de glycéryle et de 0,2 à 5,0 % en poids de chlorure de PPG-40 diéthylmonium.

7. Composition selon l'une quelconque des revendications 1 à 5, dans laquelle l'alcool comprend de 50 à 65 % en poids d'alcool dans lequel l'alcool est l'alcool éthylique, l'alcool d'isopropyle, l'alcool de n-propyle ou un mélange de ceux-ci ; le composé d'ammonium quaternaire cationique comprend de 0,01 à 0,5 % en poids de chlorure de benzalkonium et de 0,01 à 0,5 % en poids de chlorure de benzéthonium ; le système tensioactif comprend de 0,1 à 8,0 % en poids de cocamidopropyl hydroxysultaine (concentration de 50 %), de 0,2 à 5,0 % en poids de polyalkylglycoside, facultativement du laurate de glycéryle de 0,1 à 8,0 % en poids et de 0,2 à 5,0 % en poids de chlorure de PPG-40 diéthylmonium.

8. Composition selon l'une quelconque des revendications 1 à 7, dans laquelle la composition contient une quantité efficace d'un biguanide.

9. Composition selon la revendication 8, dans laquelle le biguanide est la chlorhexidine ou l'un de ses dérivés, tels que le gluconate de chlorhexidine, le gigluconate de chlorhexidine, le diacétate de chlorhexidine, le dichlorhydrate de chlorhexidine et le biguanide de polyhexaméthylène.

10. Composition selon la revendication 8, dans laquelle le biguanide est le biguanide de polyhexaméthylène, le gluconate de chlorhexidine ou un mélange de ceux-ci.

11. Composition selon l'une quelconque des revendications 8 à 10, dans laquelle le biguanide est présent dans une quantité de 0,01 à 5,0 % en poids.

12. Composition selon l'une quelconque des revendications 1 à 11, dans laquelle la composition contient une quantité efficace de système de conditionnement de la peau.

13. Composition selon la revendication 12, dans laquelle le système de conditionnement de la peau est composé de propylène glycol, de glycérine, de phényléthyldiméthicone et d'un composé quaternaire de silicone.

14. Composition selon la revendication 13, dans laquelle le propylène glycol est présent dans une quantité de 1,0 à 20 % en poids ; la glycérine dans une quantité de 1,0 à 40 % en poids ; de la phényléthyldiméthicone dans une quantité de 0,01 à 0,2 % en poids ; et le composé quaternaire de silicone dans une quantité de 0,1 à 1,0 % en poids.

15. Composition selon l'une quelconque des revendications 1 à 14, pour une utilisation dans un procédé de désinfection d'un substrat tel que la peau.
